(19) Europäisches Patentamt
European Patent Office
Office européen des brevets

(11) **EP 2 080 828 B1**

(12) **EUROPEAN PATENT SPECIFICATION**

(45) Date of publication and mention
of the grant of the patent:
**14.11.2012 Bulletin 2012/46**

(51) Int Cl.:
***D04B 15/50*** (2006.01)   ***G01N 3/08*** (2006.01)

(21) Application number: **07805855.9**

(86) International application number:
**PCT/JP2007/001000**

(22) Date of filing: **13.09.2007**

(87) International publication number:
**WO 2008/032451 (20.03.2008 Gazette 2008/12)**

(54) **METHOD OF MEASURING ELASTIC CHARACTERISTICS OF KITTING YARN AND KNITTING MACHINE**

VERFAHREN ZUR BESTIMMUNG DER ELASTISCHEN EIGENSCHAFTEN VON WIRKGARN SOWIE KOLIERWIRKMASCHINE

PROCÉDÉ DE MESURE DE PROPRIÉTÉS ÉLASTIQUES DE FIL À TRICOTER ET MACHINE À TRICOTER

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **15.09.2006 JP 2006250304**

(43) Date of publication of application:
**22.07.2009 Bulletin 2009/30**

(73) Proprietor: **Shima Seiki Mfg., Ltd
Wakayama-shi
Wakayama 641-8511 (JP)**

(72) Inventors:
• **WADA, Toshihide
Wakayama-shi, Wakayama 641-8511 (JP)**
• **YAMAGATA, Takeo
Wakayama-shi, Wakayama 641-8511 (JP)**

(74) Representative: **Wagner & Geyer
Partnerschaft
Patent- und Rechtsanwälte
Gewürzmühlstrasse 5
80538 München (DE)**

(56) References cited:
EP-A1- 1 626 113      WO-A1-2004/094712
JP-A- 03 130 443      JP-A- 06 200 453
JP-A- 08 120 548      JP-A- 2005 089 933
JP-A- 2006 118 059    JP-B2- 3 010 171
US-A- 3 067 607       US-A- 3 858 416

## Description

## Technical Field

The present invention relates to a method for measuring elastic characteristics of knitting yarn when an elastic yarn is used for forming knit fabric by a knitting machine and to a knitting machine.

## Background Art

Conventionally, an elastic yarn with large elongation percentage, also called a rubber yarn and the like, is sometimes used for the portion which requires large retractility. The elastic yarn is used in complex with other fibers that cover the surface side; with the fibers with elasticity such as polyurethane fibers, polyether ester based fibers, and others used as they are or as core fibers of covered yarn, core spun yarn, and others.

A technique to feed a rubber yarn to a knitting machine by an elastic yarn feed device equipped with a motor capable of normal-reverse rotation, and to apply tension to the rubber yarn in a direction reversal to a feed direction by reverse-rotating the motor is disclosed (see, for example, Patent Citation 1). By applying tension in the reverse direction, narrow shrunk rubber width portions can be formed in the knit fabric such as socks. In addition, a technique to partially vary gauge hand value of a knit fabric knitted using an elastic yarn as a knitting yarn and controlling supply length and yarn tension is disclosed, too (see, for example, Patent Citation 2). A construction of a knitting yarn feed device that can feed a knitting yarn to a knitting machine with desired tension is disclosed, too (see, for example, Patent Citation 3).

Patent Citation 1:Japanese Published Patent Application
JP H03-130443(2001-130443)
Patent Citation 2:Domestic re-publication
of PCT international application
No. 2004-094712(WO 2004/094712)
Patent Citation 3:United States Patent No. 3858416

## Disclosure of the Invention

## Technical Problem

As disclosed in Patent Citation 1 and Patent Citation 2, the use of an elastic yarn can vary tension at the time of knitting and can give variations to a knit fabric. However, in order to control this variation, preparatory work to seek characteristics of an elastic yarn used is needed. As necessary characteristics of the elastic yarn, there is relation of tension to elongation percentage, feed rate, or the like. In the event that there is a possibility of causing slip or yarn breakage in knitting yarn feed path, the maximum tension must be measured. However, it could be comparatively easy to determine the yarn breakage and the slip by the use of an actually used knitting machine. The relation of tension to elongation percentage or feed rate must be measured using a tension tester and the like. With a tension tester, the relation between tension and elongation is measured when a sample of predetermined length is stretched. In addition, trial knitting is performed to form knit fabric under various conditions, and based on the results, the characteristics may be determined. This kind of work requires tremendous time and skill. Furthermore, this work must be carried out every time the elastic yarn used is changed.

It is an object of this invention to provide a method for measuring elastic characteristics of knitting yarn and a knitting machine, which is capable of self-evaluating the characteristics of an elastic yarn.

## Technical Solution

This and other objects are solved by a method for self-evaluating elastic characteristics of a knitting yarn having the features and method steps, respectively, as set forth in claim 1. Preferred embodiments of this method are stated in the subclaims.

The present invention is a method for measuring elastic characteristics of a knitting yarn, with a knitting machine equipped with a yarn feeder that feeds an elastic yarn as a knitting yarn, a yarn end holding device and a yarn tension measuring device that measures the yarn tension along a feed path of the knitting yarn fed by the yarn feeder, which comprises:

varying length of a knitting yarn fed by the yarn feeder in a range from the yarn feeder to the feed path including the yarn tension measuring device; and

measuring elastic characteristics of the knitting yarn, based on a relationship between variance in the length of the knitting yarn fed in the feed path range and change in the yarn tension measured by the yarn tension measuring device.

In the method for measuring elastic characteristics of a knitting yarn according to the present invention, said yarn feeder is capable of changing over between to feed said knitting yarn to said feed path range and to return the knitting yarn from the feed path range,

the yarn feeder feeds the knitting yarn to the feed path range until the yarn tension becomes lower than a predetermined lower-limit tension after the yarn tension measuring device returns the knitting yarn from the feed path range and measures the yarn tension, and

an elongation percentage of the knitting yarn under the measured yarn tension is sought as a value derived from dividing the distance of the feed path range by a difference, which is obtained from subtracting the length of the knitting yarn fed by the yarn feeder from the distance of the feed path range.

In the method for measuring elastic characteristics of a knitting yarn according to the present invention, after elongation percentages of said knitting yarn under a plurality of the measured yarn tensions are sought, elongation percentages of the knitting yarn under different yarn tensions from the plurality of the measured yarn tensions are sought as values derived from interpolating relationship between the plurality of the yarn tensions and the plurality of the knitting yarn elongation percentages.

In the method for measuring elastic characteristics of a knitting yarn according to the present invention, while once said yarn feeder has fed said knitting yarn to said feed path range side until a yarn tension measured by said yarn tension measuring device becomes lower than said predetermined lower limit and then the yarn feeder returns the knitting yarn from the feed path range side,

said yarn tension under which said elongation percentage is sought, is determined on the occasion when the yarn tension measured by the yarn tension measuring device begins to deviate from a range predicted on the basis of a premise of elasticity characteristics.

Furthermore, the present invention is a knitting machine, as set forth in claim 5, comprising:

a yarn feeder that feeds an elastic yarn as a knitting yarn and can detect the length to be fed;
a yarn end holding device;
a yarn tension measuring device that measures the yarn tension of the knitting yarn along a path through which the knitting yarn is fed; and
a controller that controls the yarn feeder and yarn tension measuring device,
wherein the controller sets the range of the feed path, and controls in such a manner to execute the method for measuring elasticity characteristics of a knitting yarn.

## Advantageous Effects

According to the present invention, because the elastic characteristics of a knitting yarn are measured based on a relationship between variance in length of the knitting yarn fed in feed path range and change in yarn tension measured by the yarn tension measuring device when the knitting yarn is sent out from or returned to a yarn feeder to the feed path range, along which the yarn tension measuring device is installed, it is possible to measure the elastic characteristics of the knitting yarn using a knitting machine. It is possible to measure elastic characteristics of a knitting yarn and to self-evaluate using a knitting machine used for knitting fabric, measurement of the elastic characteristics as a preparatory work is no longer required, and it becomes possible to comprehend elastic characteristics of the knitting yarn and to form highly accurate knit fabric.

In addition, according to the present invention, the yarn feeder feeds out a knitting yarn with no yarn tension to the feed path range side to which the yarn tension is applied, but the length of the knitting yarn to be fed out can be measured with no yarn tension. At the moment when the yarn tension in the feed path range side becomes lower than the predetermined lower limit, the length of the knitting yarn with no yarn tension becomes nearly equal to the distance of the feed path range to which the knitting yarn is fed out from the yarn feeder. The measured length of the knitting yarn to be fed out until the yarn tension decreases below the lower limit is equivalent to the elongation that corresponds to the yarn tension when the knitting yarn begins to be fed out from the yarn feeder. Because the difference between the distance of the feed path range and the length of the measured knitting yarn is the length in the vicinity of zero yarn tension of the knitting yarn existing under the yarn tension when the yarn begins to be fed out, it is possible to easily compute the elongation percentage by dividing the distance by this length.

According to the present invention, even if elongation that corresponds to a yarn tension of various values is not measured, the correspondence relation between the yarn tension and the elongation percentage can be computed by interpolation.

Furthermore, according to the present invention, by the use of a knitting machine, the elastic characteristics of a knitting yarn can be automatically self-evaluated.

**Brief Description of Drawings**

[Fig. 1] Fig. 1 is a simplified block diagram showing construction of a flat knitting machine 1 that can execute a method for measuring elastic characteristics of a knitting yarn as one embodiment of the present invention.

[Fig. 2] Fig. 2 is a graph showing one example of a change over time about the yarn tension when the yarn end of a knitting yarn 5 is held by a gripper 7 and a yarn feed roller 16 is reversed in the flat knitting machine 1 of Fig. 1.

[Fig. 3] Fig. 3 is a graph showing one example of a change over time about the yarn tension when the yarn feed roller 16 is rotated in the reverse direction and then the yarn feed roller 16 is rotated in the normal direction.

[Fig. 4] Fig. 4 is a flow chart showing schematic procedures of a method for measuring elastic characteristics of a knitting yarn as one embodiment of the present invention.

[Fig. 5] Fig. 5 is a graph showing temporal change of the yarn tension T when elastic characteristics of the knitting yarn 5 are measured in accordance with Fig. 4.

[Fig. 6] Fig. 6 is a graph showing the concept of interpolating the relation between the yarn tension and the elongation percentage in Step s11 of Fig. 4.

**Explanation of Reference**

**[0017]** -

1     Flatknitting machine
2     Main body of the knitting machine
3     Yarn feed device
4     Needle beds
5     Knitting yarn

6     Carrier

7     Gripper

10    Control unit
11    Input section
12    Knit operation control section

13    Yarn feed control section

14    Memory section

15    Yarn cone

16    Yarn feed roller

17    Tension meter

**Best Mode for Carrying Out the Invention**

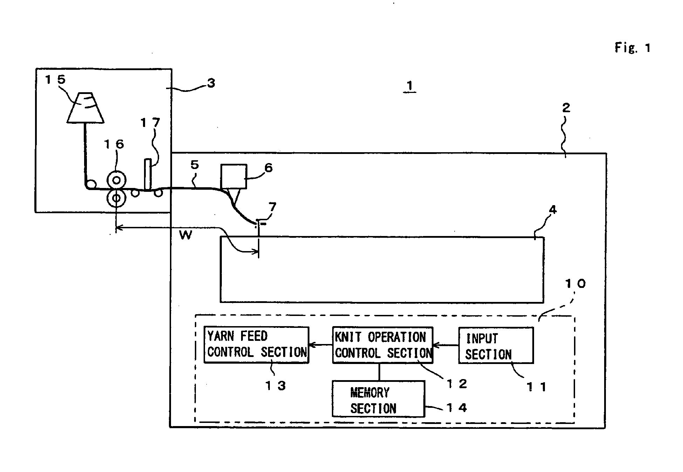

Fig. 1 is a simplified block diagram showing construction of a flat knitting machine 1 that can execute a method for measuring elastic characteristics of a knitting yarn as one embodiment of the present invention. The flat knitting machine 1 is schematically formed with a main body of the knitting machine 2 and a yarn feed device 3. In the main body of the knitting machine 2, needle beds 4 equipped with a large number of knitting needles are included and a knitting yarn 5 is fed to knitting needles, and when knitting action of the knitting needles is carried out, a knit fabric is formed. The knitting yarn 5 is fed from the yarn feed device 3 to the knitting needles which carry out knitting action through a feed path via a carrier 6. When predetermined knit fabric is formed, the knitting yarn 5 is cut and the head end of the knitting yarn 5 is held by a gripper 7 until forming of the next knit fabric begins. The applicant of the present invention has disclosed a construction of the gripper 7 as a yarn end holding device, in, for example, Japanese Published Patent Application No. 2005-089933 and others.

In the main body of the knitting machine 2, a control device 10 is installed, too. In the control device 10, an input section 11, knit operation control section 12, yarn feed control section 13, and memory section 14 are included. To the input section 11, instructions from an operator of the flat knitting machine 1, data of knit fabric to be formed, and others are input. Input is carried out by operation of a keyboard and the like, data communication, or mounting of storage media including USB memory and others. The knit operation control section 12 controls each unit of the main body of the knitting machine 2 and conducts formation of knit fabric. The yarn feed control section 13 controls a yarn feed roller 16 and the like of the yarn feed device 3 and allows the yarn feed device 3 to feed the knitting yarn 5. In the memory section 14, data of knit fabric to be formed and others are stored, and actual achievement data records such as production conditions are accumulated.

The knitting yarn 5 is fed to the main body of the knitting machine 2 by the yarn feed device 3. In the yarn feed device 3 that can feed the knitting yarn 5 from a yarn cone 15, the yarn feed roller 16 is installed. Rotating the yarn feed roller 16 in the normal direction dispatches the knitting yarn 5 and reversing the yarn feed roller 16 returns the knitting yarn 5. The yarn feed roller 16, as a yarn feed device, rotation rate corresponds to feed length of the knitting yarn 5 within a range in which slipping of the yarn does not occur. Reversing the yarn feed roller 16 with the head end of the knitting yarn 5 held by the gripper 7 or with the knitting yarn 5 knitted in knit fabric locked to the needle bed 4 can increase the yarn tension of the knitting yarn 5 between the yarn feed roller 16 and the gripper 7 or the knit fabric. Even when the knitting yarn 5 is consumed by knitting the knit fabric, or when normal rotation of the yarn feed roller 16 delays, the yarn tension increases. In the event that the yarn feed roller 16 rotates in a normal direction and feeds the knitting yarn 5 in excess, the yarn tension of the knitting yarn 5 decreases. The yarn tension can be measured by a tension meter 17.

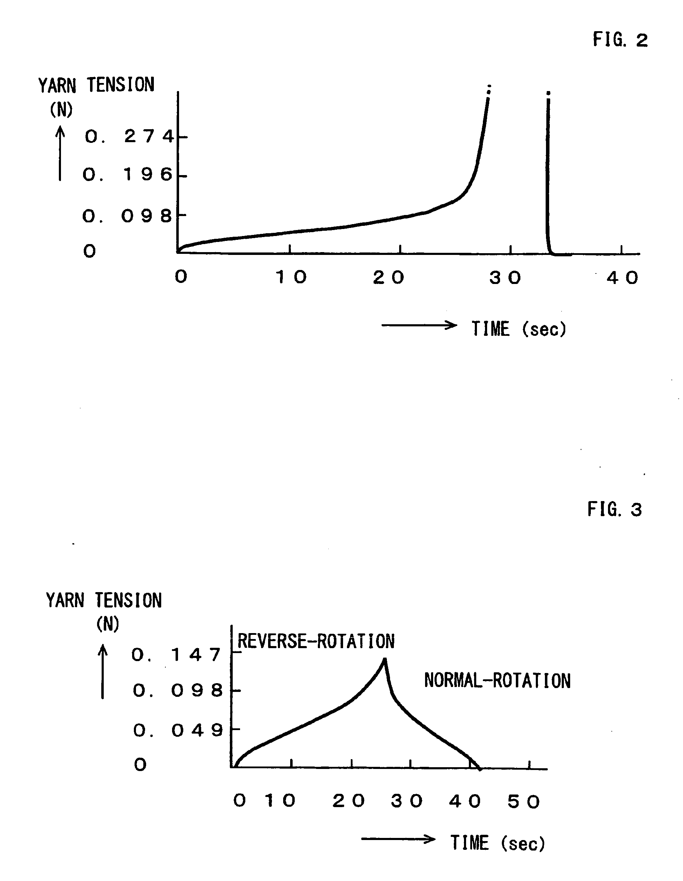

Fig. 2 shows one example of a change over time about the yarn tension experimentally measured assuming the case in which the yarn end of the knitting yarn 5 is held by the gripper 7 and the yarn feed roller 16 is reversed at a fixed angular speed in the flat knitting machine 1 of Fig. 1. However, as a matter of convenience of a test, a yarn end holding device that corresponds to the gripper 7 without going through the carrier 6 from the yarn feed roller 16 is disposed so that the free length W becomes 100 mm. Between the yarn feed roller 16 and the yarn end holding device, the tension meter 17 is installed. In the actual flat knitting machine 1, the free length W of the knitting yarn 5 from the yarn feed roller 16 to the gripper 7 when the yarn tension of the knitting yarn 5 is zero is about 1 meter. Though the rotating speed of the yarn feed roller 16 is low, the yarn tension rapidly increases when 25 seconds pass from the rotation start. The yarn tension exceeds the upper limit that can be measured by the tension meter 17, the knitting yarn 5 is cut in the vicinity of 33 seconds, and the yarn tension returns to zero.

Fig. 3 shows one example of a temporal change about the yarn tension in a test, where the free length W is set to 190 mm for the knitting yarn 5 shown in Fig. 1 and the yarn feed roller 16 is rotated in the normal direction until the yarn tension becomes lower than the lower-limit value in the vicinity of zero after the yarn feed roller 16 is reversed until the yarn tension reaches 0.147 N (15 gf). The change in the yarn tension is assumed to have a large error in a range of a small yarn tension and except the range of this large error, the change in the yarn tension seems to have a resemblance to changes of exponential functions. The length of the knitting yarn 5 fed by rotating the yarn feed roller 16 in a normal direction until the yarn tension becomes below the lower limit value in the vicinity of zero yarn tension corresponds to the length on the side of the yarn cone 15 of the yarn feed roller 16. In the event that the yarn tension on the yarn cone 15 side is nearly zero, the length of the knitting yarn 5 fed from the yarn feed roller 16 is the length in the free-length state. Let d denote the length of the knitting yarn 5 fed while the yarn tension is between 0.147N and zero; then, it is clear that when the yarn tension is 1.47N, in the section of distance W from the yarn feed roller 16 to the gripper 7, the knitting yarn 5 of the free length W-d exists, and the elongation percentage is W / (W-d) x 100%.

Reversing the yarn feed roller 16 with a fixed distance W held, as shown in Fig. 1, elongates the knitting yarn 6 and the free length of the knitting yarn 5 that occupies the distance W under the increased yarn tension becomes smaller than W. In the range in which an elastic yarn elastically stretches, it is assumed that elongation $\Delta W$ and the yarn tension T is proportional. That is, with k designated as a coefficient of proportion, the following Eq. (1) is evolved.

$$\Delta W = k \times T \qquad \qquad \dots (1)$$

At the yarn tension T, the knitting yarn 5 of the free length W stretches in the length of W + $\Delta W$, and the free length of the knitting yarn 5 that exists between the yarn feed roller 16 and the gripper 7 of Fig. 1 can be given by the following Eq. (2).

$$W \times (W / (W + \Delta W)) = W / (1 + (k \times T) / W) \qquad \dots (2)$$

When the yarn feed roller 16 reverses only by a very little time Δt, the knitting yarn 5 under the yarn tension T is elongated by just r x ω x Δt where r denotes the radius of the yarn feed roller 16 and ω the angular speed. Because the knitting yarn 5 in the state of Eq. (2) stretches, let ΔT denote an increment of the yarn tension T caused by this; then, the following Eq. (3) is obtained.

$$r \times \omega \times \Delta t \times W / (1 + (k \times T) / \ \ W) = k \times \Delta T \qquad \ldots (3)$$

Ordering Eq. (3), the following Eq. (4) is obtained where a and ß are constants.

$$\Delta t = (\alpha \times \Delta T) \ / \ (1 + \beta \times T) \qquad \ldots (4)$$

Integrating both sides of Eq. (4), the following Eq. (5) is obtained where C is constant:

$$t = (\alpha / \beta) \times \ln (1 + \beta \times T) + C \qquad \ldots (5).$$

From Eq. (5), a change for time t of the yarn tension T is found as shown in the following Eq. (6);

$$T = a \times (\exp (t - b) - c) \qquad \ldots (6).$$

where, a, b, and c are constants.

From Fig. 2 and Fig. 3, too, in the range where the yarn tension is large, it is assumed that the yarn tension changes in accordance with exponential function as shown in Eq. (6). In the event that the tension meter 17 is to measure a yarn tension in such a manner that a depression caused by a load applied to a yarn in a predetermined section corresponds to the yarn tension, the measurement accuracy lowers and it is assumed that the yarn tension would not change in accordance with the exponential function in the range where the yarn tension is small.

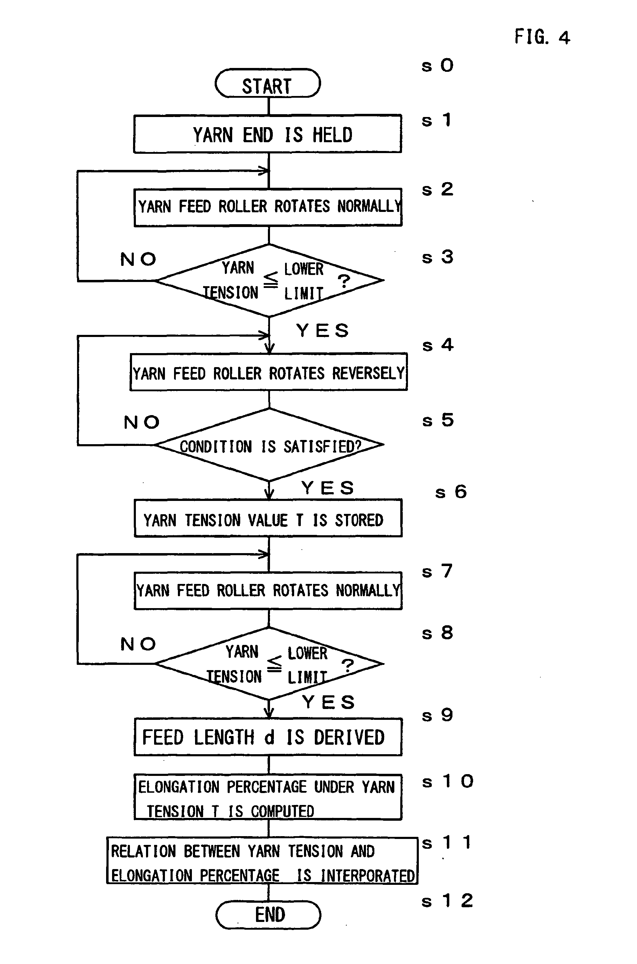

Fig. 4 shows schematic procedures of a method for measuring elastic characteristics of a knitting yarn as one embodiment of the present invention. The procedure starts from Step s0, and in Step s1, the yarn end of the knitting yarn 5 is held by the gripper 7 as shown in Fig 1. In Step s2, the yarn feed roller 16 is rotated in a normal direction. In Step s3, whether or not the yarn tension becomes below the lower limit is confirmed. In principle, it is desirable to confirm whether or not the yarn tension becomes zero but in actuality, in the range where the yarn tension becomes near zero and is small, the measurement accuracy of the yarn tension lowers; therefore, the lower limit is set in advance. In addition, with the yarn end of the knitting yarn 5 held by the gripper 7, there is a possibility that a small yarn tension may be applied. When the yarn tension reaches this kind of small value, it is assumed that the knitting yarn 5 acquires nearly free length. If the yarn tension is not lower than the lower limit, normal rotation of Step s2 continues. That is, after holding the yarn end by the gripper 7, the yarn feed roller 16 is rotated in the normal direction until the yarn tension becomes lower than the lower limit.

When it is confirmed in Step s3 that the yarn tension becomes lower than the lower limit, the yarn feed roller 16 is reversed and the knitting yarn 5 is returned in Step s4. Since the knitting yarn 5 is returned from the section of the distance W between the yarn feed roller 16 and the gripper 7 to the yarn cone 16 side, the knitting yarn 5 stretches and the yarn tension rises on the gripper 7 side. For the knitting yarn 5, the reference tension set on the basis of the range of the yarn tension used at the time of knitting knit fabric, or usable maximum tension, and other upper-limit conditions for measuring the yarn tension must be set in advance. In Step s5, whether or not the yarn tension satisfies the conditions is determined. If the yarn tension does not satisfy the conditions, the procedure returns to Step s4. In the event that the maximum tension is set as a condition, for example, if a deviation from the range predicted for the yarn tension to change in accordance with the exponential function exceeds a predetermined reference, the tension is judged as the maximum tension and the conditions of Step s5 are assumed to be satisfied. In this way, by reversing the yarn feed roller 16, the maximum tension is able to be found from the yarn tension changing condition. The yarn tension value T of the maximum tension found and the like are stored in the memory section 14 of Fig. 1 in Step s6.

In Step s7, the yarn feed roller 16 is rotated again in the normal direction and it is confirmed in Step s8 that the yarn tension becomes below the lower limit. The rotating amount of the yarn feed roller 16 is stored in the memory section. The yarn feed roller 16 continues normal rotation of Step s7 until the yarn tension becomes lower than the lower limit in Step s8. When the yarn tension becomes lower than the lower limit in Step s8, the feed length d of the knitting yarn 5 is computed in accordance with the rotation amount of the yarn feed roller 16 in Step s9. As described above, by keeping the yarn tension on the yarn cone 15 side of the yarn feed roller 16 in the vicinity of zero, the feed length d can be directly found from the rotation amount. Incidentally, in the event that the yarn feed roller 16 rotates at constant speed, the rotation amount is proportional to the rotating time. Therefore, the time is measured and the feed length d can be derived. However, in the event that the rotating speed greatly changes, including acceleration at the time of start and deceleration at the time of stop, it is desirable to directly find the rotating amount by a method for detecting the angular displacement of the rotating axis of the yarn feed roller 16 by encoder or the like.

In Step s10, the free length of the knitting yarn 5 existing between the yarn feed roller 16 and the gripper 7 at the yarn tension value T stored in the memory section in Step s6 is computed to be W - d, and the elongation percentage is computed to be W / (W - d) %, and the relation between the yarn tension and the elongation percentage as well as feed length is determined. In Step s11, based on the computed value of the elongation percentage at the yarn tension value T, the elongation percentage at other yarn tension is derived from interpolation. In Step s12, the procedures of measuring elastic characteristics of the knitting yarn are completed.

The foregoing measurement procedures are able to be automatically conducted by setting a program in advance in the control unit 10 of Fig. 1, except the procedure of holding the yarn end in Step s1. Incidentally, though the yarn end of the knitting yarn 5 is held by the use of the gripper 7, the gripper 7 might be set to hold the middle of the knitting yarn 5 from knit fabric to the yarn feed roller 16. In addition, the knitting yarn 5 may be hooked to knitting needles with care to prevent it from coming off during testing, or retention may be conducted by the formed knit fabric itself to measure elastic characteristics. Furthermore, a supporting device specialized for elastic characteristics measurement may be installed.

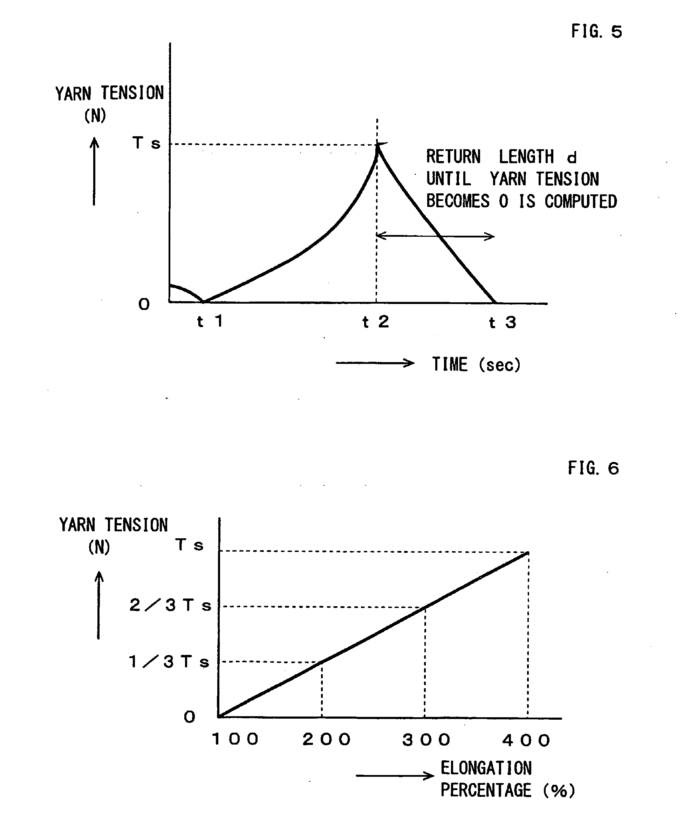

Fig. 5 shows temporal changes in yarn tension T when elastic characteristics of the knitting yarn 5 are measured according to Fig. 4. Since it is premised on rotation of the yarn feed roller 16 at a constant speed, the yarn tension T changes equivalently even when the feed length change is used in place of time change. To time t1, the yarn feed roller 16 is rotated in the normal direction and the yarn tension is brought to be lower than the lower limit in the vicinity of zero in conformity to the procedures from Step s1 to Step s3 of Fig. 4. From time t1 to t2, in conformity to the procedures from Step s4 to Step s6 of Fig. 4, the yarn feed roller 16 is reversed and the yarn tension Ts that satisfies certain conditions. From time t2 to t3, in conformity to the procedures from Step s7 to Step s9 of Fig. 4, the yarn feed roller 16 is rotated in the normal direction and the return length d until the yarn tension becomes lower than the lower limit is computed.

Incidentally, the return length d is able to be found from changes in yarn tension from time t1 to t2. In the event that a change in yarn tension in this time range accurately conforms to a predetermined function such as an exponential function, based on the time of t2-t1 and the angular speed $\omega$ and the radius r of the yarn feed roller 16, the return length d is able to be found by the functional calculus as the length which the yarn feed roller 16 feeds from the gripper 7 side to the yarn cone 15 side. It is also possible to actually measure the return length d by installing a length measuring roller, and the like on the yarn cone 15 side of the yarn feed roller 16. In addition, for a plurality of yarn tensions between time t2 and time t3 of Fig. 5, from the return rates that correspond to relevant yarn tensions, the elongation percentage can be sought, respectively.

Fig. 6 shows a concept of interpolating the relation between the yarn tension and the elongation percentage in Step s11 of Fig. 4 on the supposition that the elongation percentage at the yarn tension Ts obtained in Fig. 5 is 400%. Since the elongation percentage when the yarn tension is zero is 100%, the elongation percentage is assumed to be proportional to the yarn tension between the 100% elongation percentage and the 400% elongation percentage. That is, when the yarn tension becomes 1 / 3 of the Ts, the elongation percentage becomes 200%, and when the yarn tension becomes 2 / 3 of the Ts, the elongation percentage becomes 300%. Let the feed length of the knitting yarn 5 be one needle equivalence at the 100% elongation percentage; then, at the 200% elongation percentage at yarn tension of 1 / 3 Ts, the feed length becomes 1 / 2 needle equivalence, and at the 400% elongation percentage, the feed length of the knitting yarn 5 becomes 1 / 4 needle equivalence.

In the event that the elongation percentages are sought for a plurality of yarn tensions, respectively, the data is plotted on a graph to find the relation between the yarn tensions and the elongation percentages. The elongation percentage for other yarn tensions can be found by interpolating the relation obtained as a graph.

Incidentally, the present invention is able to be suitably applied to a case when for example, tubular-form knit fabric is formed in a taper form by the flat knitting machine 1 as shown in Fig. 1. The taper-form portion is formed by gradually varying the tension of the elastic yarn, and reproducibility of the shape can be improved by using the knitted yarn 5 with the relationship between the yarn tension and the elongation percentage identified. Furthermore, the present invention is able to be applied not only to cases in which an elastic yarn is used for the knitting yarn 5 but also generally to cases in which the elastic characteristics of a knitting yarn must be identified by a knitting machine that forms knit fabric.

**EP 2 080 828 B1**

**Claims**

1. A method for self-evaluating elastic characteristics of a knitting yarn (5), with a knitting machine (1) equipped with a yarn feeder (3) that feeds an elastic yarn as a knitting yarn (5), a yarn end holding device (7) and a yarn tension measuring device (17), installed between the yarn feeder (3) and the yarn end holding device (7), that measures the yarn tension along a feed path of the knitting yarn (5) fed by the yarn feeder (3), which comprises:

   holding an end of the knitting yarn (5) by the yarn end holding device (7);
   varying the length of the knitting yarn (5), fed by the yarn feeder (3), in the feed path range, along which the yarn tension measuring device (17) is installed; and
   evaluating elastic characteristics of the knitting yarn (5), based on a relationship between the variance in the length of the knitting yarn (5) fed in the feed path range and change in the yarn tension measured by the yarn tension measuring device (17).

2. The method for self-evaluating elastic characteristics of a knitting yarn according to claim 1, wherein said yarn feeder (3) is capable of changing over between to feed said knitting yarn (5) to said feed path range and to return the knitting yarn (5) from the feed path range and wherein the yarn feeder (3) returns the knitting yarn (5) from the feed path range and the yarn tension measuring device (17) measures the yarn tension T;
   whereafter the yarn feeder (3) feeds the knitting yarn (5) to the feed path range until the yarn tension becomes lower than a predetermined lower-limit tension; and
   an elongation percentage of the knitting yarn (5) under the measured yarn tension T is sought as a value derived from dividing the distance of the feed path range by a difference, which is obtained from subtracting the length of the knitting yarn (5) fed by the yarn feeder (3) from the distance of the feed path range.

3. The method for self-evaluating elastic characteristics of a knitting yarn according to claim 2, wherein after elongation percentages of said knitting yarn (5) under a plurality of the measured yarn tensions are sought, elongation percentages of the knitting yarn (5) under yarn tensions different from the plurality of measured yarn tensions are sought as values derived from interpolating the relationship between the plurality of measured yarn tensions and the plurality of sought knitting yarn elongation percentages.

4. The method for self-evaluating elastic characteristics of a knitting yarn according to claims 2 or 3, wherein said measured yarn tension T is a maximum tension, which can be applied and which is determined on the occasion when the yarn tension measuring device (17) measures a yarn tension deviating from a range predicted on the basis of a premise of elasticity characteristics, while said yarn feeder (3) returns the knitting yarn (5) from the feed path range side.

5. A knitting machine (1), comprising:

   a yarn feeder (3) that feeds an elastic yarn as a knitting yarn (5) and can detect the length to be fed;
   a yarn end holding device (7);
   a yarn tension measuring device (17), installed between the yarn feeder (3) and the yarn end holding device (7), that can measure the yarn tension of the knitting yarn (5) along a path through which the knitting yarn (5) is fed; and
   a controller (10) that can control the yarn feeder (3) and yarn tension measuring device (17),
   wherein the controller (10) is meant to set the range of the feed path, and to control the yarn feeder (3) and yarn tension measuring device (17) in such a manner as to execute the method for self-evaluating elastic characteristics of a knitting yarn according to any one of claims 1-4.

**Patentansprüche**

1. Verfahren zum Bestimmen der elastischen Eigenschaften eines Strickgarns (5) mit einer Strickmaschine (1), die ausgerüstet ist mit einem Fadenführer (3), der ein elastisches Garn als Strickgarn (5) führt, einer Garnende-Halteeinrichtung (7) und

eine Garnspannungs-Messeinrichtung (17), die zwischen dem Fadenführer (3) und der Garnende-Halteeinrichtung (7) angeordnet ist, und die die Garnspannung entlang eines Fadenwegs des Strickgarns (5) misst, das vom Fadenführer (3) geführt ist, mit folgenden Verfahrensschritten:

Halten eines Endes des Strickgarns (5) durch die Garnende-Halteeinrichtung (7);
Ändern der Länge des Strickgarns (5), das vom Fadenführer (3) geführt wird, im Fadenwegbereich entlang dem die Garnspannungs-Messeinrichtung (17) installiert ist; und
Bewerten der elastischen Eigenschaft des Strickgarns (5) basierend auf einem Zusammenhang zwischen der Längenänderung des Strickgarns (5), das im Garnwegbereich geführt ist, und der Änderung in der Garnspannung, die von der Garnspannungs-Messeinrichtung (17) gemessen wird.

2. Verfahren zum Bestimmen der elastischen Eigenschaften eines Strickgarns nach Anspruch 1, wobei der Fadenführer (3) zwischen dem Führen des Strickgarns (5) zum Fadenwegbereich und der Umkehr des Strickfadens (5) vom Fadenwegbereich umschalten kann, und wobei der Fadenführer (3) das Strickgarn (5) vom Fadenwegbereich umkehrt und die Fadenspannungsmesseinrichtung (17) die Fadenspannung T misst;
wobei danach der Fadenführer (3) das Strickgarn (5) zum Fadenwegbereich führt bis die Garnspannung kleiner als eine vorgegebene untere Grenzspannung wird; und
ein Dehnungs-Prozentsatz des Strickgarns (5) unter der gemessenen Garnspannung (T) als Wert gesucht wird, der durch Dividieren des Abstandes des Fadenwegbereichs durch eine Differenz abgeleitet wird, die durch Abziehen der Länge des Strickgarns (5), das vom Fadenführer (3) geführt wird, vom Abstand des Fadenführerwegs erhalten wird.

3. Verfahren zum Bestimmen der elastischen Eigenschaften eines Strickgarns nach Anspruch 2, wobei nachdem die Dehnungs-Prozentsätze des Strickgarns (5) unter mehreren gemessenen Garnspannungen gesucht wurde, die Dehnungsprozentsätze des Strickgarns (5) unter den Garnspannungen, die von den mehreren gemessenen Garnspannungen unterschiedlich sind, als Werte gesucht werden, die durch Interpolation des Zusammenhangs zwischen den mehreren gemessenen Garnspannungen und den mehreren gesuchten Strickgarn-Dehnungs-Prozentsätzen abgeleitet werden.

4. Verfahren zum Bestimmen der elastischen Eigenschaften eines Strickgarns nach Anspruch 2 oder 3, wobei die gemessene Garnspannung T eine maximale Spannung ist, die angewandt werden kann, und die dann bestimmt wird, wenn die Garnspannungs-Messeinrichtung (7) eine Garnspannung misst, welche von einem Bereich abgeleitet ist, der auf der Basis einer Vorhersage der Elastizitätseigenschaften vorhergesagt wurde, während der Fadenführer (3) das Strickgarn (5) von der Zuführungswegbereichsseite zurückführt.

5. Strickmaschine (1) mit
einem Fadenführer (3), der ein elastisches Garn als Strickgarn (5) zuführt und die zuzuführende Länge ermitteln kann;
eine Garnende-Halteeinrichtung (7);
eine Garnspannungs-Messeinrichtung (17), die zwischen dem Fadenführer (3) und der Garnende-Halteeinrichtung (7) vorgesehen ist und die die Garnspannung des Strickgarns (5) entlang eines Weges messen kann, über den das Strickgarn (5) zugeführt ist, und
einer Steuereinrichtung (10), die den Fadenführer (3) und die Garnspannungs-Meßeinrichtung (17) steuern bzw. regeln kann,
wobei die Steuereinrichtung (10) dafür bestimmt ist, den Bereich des Zuführungsweg einzustellen, und den Fadenführer (3) und die Fadenspannungs-Meßeinrichtung (17) in der Weise zu Steuern, um das Verfahren zur Bestimmung der elastischen Eigenschaften eines Strickgarns gemäß einem der Ansprüche 1 bis 4 auszuführen.

**Revendications**

1. Procédé d'auto-évaluation de caractéristiques élastiques d'un fil à tricoter (5), à l'aide d'une machine à tricoter (1) équipée
d'un dispositif d'alimentation en fil (3) qui fournit un fil élastique en tant que fil à tricoter (5),
d'un dispositif de maintien d'extrémité de fil (7),
et d'un dispositif de mesure de tension de fil (17), installé entre le dispositif d'alimentation en fil (3) et le dispositif de maintien d'extrémité de fil (7), qui mesure la tension du fil le long d'un chemin d'alimentation du fil à tricoter (5) fourni par le dispositif d'alimentation en fil (3),
le procédé comprenant les étapes suivantes :

maintenir une extrémité du fil à tricoter (5) par le dispositif de maintien d'extrémité de fil (7) ;

faire varier la longueur du fil à tricoter (5), fourni par le dispositif d'alimentation en fil (3), dans la plage du chemin d'alimentation le long duquel le dispositif de mesure de tension de fil (17) est installé ; et

évaluer des caractéristiques élastiques du fil à tricoter (5), sur la base d'une relation entre la variation de la longueur du fil à tricoter (5) fournie dans la plage du chemin d'alimentation et la variation de la tension du fil mesurée par le dispositif de mesure de tension de fil (17).

2. Procédé d'auto-évaluation de caractéristiques élastiques d'un fil à tricoter selon la revendication 1, dans lequel le dispositif d'alimentation en fil (3) peut changer entre le fait de fournir le fil à tricoter (5) à la plage du chemin d'alimentation et le fait de ramener le fil à tricoter (5) à partir de la plage du chemin d'alimentation, et dans lequel le dispositif d'alimentation en fil (3) ramène le fil à tricoter (5) à partir de la plage du chemin d'alimentation et le dispositif de mesure de tension de fil (17) mesure la tension T du fil ;

puis le dispositif d'alimentation en fil (3) fournit le fil à tricoter (5) à la plage du chemin d'alimentation jusqu'à ce que la tension du fil devienne inférieure à une tension limite inférieure prédéterminée ; et

un pourcentage d'allongement du fil à tricoter (5) sous la tension de fil T mesurée est déterminé en tant que valeur déduite de la division de la distance de la plage du chemin d'alimentation par une différence qui est obtenue en soustrayant la longueur de fil à tricoter (5) fournie par le dispositif d'alimentation en fil (3) de la distance de la plage du chemin d'alimentation.

3. Procédé d'auto-évaluation de caractéristiques élastiques d'un fil à tricoter selon la revendication 2, dans lequel après avoir déterminé des pourcentages d'allongement du fil à tricoter (5) sous une pluralité de tensions de fil mesurées,

des pourcentages d'allongement du fil à tricoter (5), sous des tensions de fil différentes de la pluralité de tensions de fil mesurées, sont déterminés en tant que valeurs déduites de l'interpolation de la relation entre la pluralité de tensions de fil mesurées et la pluralité de pourcentages d'allongement de fil à tricoter déterminés.

4. Procédé d'auto-évaluation de caractéristiques élastiques d'un fil à tricoter selon la revendication 2 ou 3, dans lequel la tension T de fil mesurée est une tension maximum qui peut être appliquée et qui est déterminée dans le cas où le dispositif de mesure de tension de fil (17) mesure une tension de fil s'écartant d'une plage prédite sur la base d'une proposition de caractéristiques élastiques, tandis que le dispositif d'alimentation en fil (3) ramène le fil à tricoter (5) à partir du côté de la plage du chemin d'alimentation.

5. Machine à tricoter (1), comprenant :

un dispositif d'alimentation en fil (3) qui fournit un fil élastique en tant que fil à tricoter (5) et peut détecter la longueur à fournir ;

un dispositif de maintien d'extrémité de fil (7) ;

un dispositif de mesure de tension de fil (17), installé entre le dispositif d'alimentation en fil (3) et le dispositif de maintien d'extrémité de fil (7), qui peut mesurer la tension du fil à tricoter (5) le long d'un chemin par lequel le fil à tricoter (5) est fourni ; et

un contrôleur (10) qui peut contrôler le dispositif d'alimentation en fil (3) et le dispositif de mesure de tension de fil (17),

le contrôleur (10) étant destiné à régler la plage du chemin d'alimentation, et à contrôler le dispositif d'alimentation en fil (3) et le dispositif de mesure de tension de fil (17) de façon à exécuter le procédé d'auto-évaluation de caractéristiques élastiques d'un fil à tricoter selon l'une quelconque des revendications 1 à 4.

Fig. 1

FIG. 2

YARN TENSION
(N)

FIG. 3

YARN TENSION
(N)

FIG. 4

START — s0

YARN END IS HELD — s1

YARN FEED ROLLER ROTATES NORMALLY — s2

$\text{YARN TENSION} \leqq \text{LOWER LIMIT}$ ? — s3
NO / YES

YARN FEED ROLLER ROTATES REVERSELY — s4

CONDITION IS SATISFIED? — s5
NO / YES

YARN TENSION VALUE T IS STORED — s6

YARN FEED ROLLER ROTATES NORMALLY — s7

$\text{YARN TENSION} \leqq \text{LOWER LIMIT}$ ? — s8
NO / YES

FEED LENGTH d IS DERIVED — s9

ELONGATION PERCENTAGE UNDER YARN TENSION T IS COMPUTED — s10

RELATION BETWEEN YARN TENSION AND ELONGATION PERCENTAGE IS INTERPORATED — s11

END — s12

13

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H03130443 B
- WO 2001130443 A
- WO 2004094712 PCT
- WO 2004094712 A
- US 3858416 A
- JP 2005089933 A